# EUROPEAN PATENT APPLICATION

(11) **EP 2 824 096 A1**
(43) Date of publication of application: **14.01.2015**
(21) Application number: 13175668.6
(22) Date of filing: 09.07.2013
(51) Int. Cl.: C07C 68/02, C07C 69/96, H01M 6/16

(54) **Fluorinated carbonates comprising double bond-containing groups, methods for their manufacture and uses thereof**

(71) Applicant: Solvay SA, 1120 Bruxelles (BE)
(72) Inventor: The designation of the inventor has not yet been filed
(74) Representative: Mross, Stefan P.M.

(57) **Abstract**

Fluorinated carbonates comprising double-bond containing groups were prepared and their use as solvent additives or solvents in lithium ion batteries, lithium air batteries, lithium sulphur batteries and supercapacitors is described. Preferred compounds contain at least one alkenyl or at least one (hetero)aryl group.

## Description

The present invention concerns fluorinated carbonates comprising double bond-containing groups, methods for the preparation thereof, and their use as solvent or solvent additive for lithium ion batteries and supercapacitors.

Lithium ion batteries, lithium air batteries and lithium sulfur batteries are well-known rechargeable means for storing electric energy. Lithium ion batteries comprise an electrolyte composition containing a solvent, a conductive salt and, often, additives. The solvent is an aprotic organic solvent which serves to dissolve the conductive salt. See, for example, WO 2007/042471 which provides information concerning suitable solvents. Suitable conductive salts are known in the art. LiPF₆ is a preferred conductive salt.

Capacitors are widely used devices for storing electrical energy. Among the various types of capacitors are electrochemical capacitors and electrolytic capacitors.

A hybrid supercapacitor is an electrochemical energy storage device that employs two different electrode types, the difference between the electrodes generally being in capacity or composition, and an electrolyte composition.

The optimization of the electrolyte compositions in hybrid supercapacitors still offers a significant potential to improve the performance properties of such systems.

Additives improve the properties of lithium ion batteries, e.g. by extending the cycle life. Fluoroalkyl alkyl carbonates, e.g. fluoromethyl methyl carbonate, and carbamates are known solvent additives for lithium ion batteries. WO 2011/006822 discloses the manufacture of 1-fluoroalkyl (fluoro)alkyl carbonates and carbamates.

The objective of the present invention is to provide improved additives for lithium ion batteries, lithium air batteries, lithium sulphur batteries or supercapacitors. The compounds of the present invention provide advantages like modifying the viscosity or reducing the flammability. Another advantage is the modification of the electrodes under formation of beneficial films. Furthermore, the compounds of the invention lead to a better wettability of materials used in lithium ion batteries such as in particular a separator. The compounds of the invention can suitably assist in the protection against overcharging, for example, by serving as a redox shuttle. Yet another advantage is an increase in stability of the electrolyte composition, e.g. in presence of copper anions, which can be formed by possible degradation of certain current collector materials.

Additionally, the compounds of the present invention may increase energy density of a supercapacitor, their power density or their cycle life.

One aspect of the invention concerns a compound of the general formula R¹R²CF-O-C(O)-O-R³ wherein R¹ and R² are independently H, F, alkyl, cycloalkyl, alkylene-aryl, or alkylene-heteroaryl ; and wherein R³ is a double bond-containing group.

The term "double bond-containing group" is intended to denote a group wherein at least two atoms are bonded together chemically by means of a double bond. Thus, the double bond-containing groups encompass alkenyl groups as well as aryl and heteroaryl groups. Specific examples of alkenyl include vinyl, allyl and 1-butenyl. The aryl groups are derived from an aromatic nucleus such as, in particular, a C6-C10 aromatic nucleus, in particular phenyl or naphthyl. The heteroaryl groups are derived from an aromatic nucleus wherein at least one atom in the nucleus is a heteroatom ; preferably the at least one heteroatom is O, S, or N. Specific examples of heteroaryl groups are thiophene, furan, triazole, pyrazole, pyridine, pyrimidine, oxazole, thiazole, and isoxazole.

The term "alkyl group" is intended to denote an optionally substituted chain of saturated hydrocarbon-based groups, such as, in particular, a C1-C6 alkyl. By way of example, mention may be made of methyl, ethyl, propyl, isopropyl, butyl, t-butyl, pentyl, isopentyl and hexyl. The alkyl may be optionally substituted, e.g. with halogen, aryl, or heteroaryl.

The term "cycloalkyl group" is intended to denote an optionally substituted cycle of saturated hydrocarbon-based groups. By way of example, mention may be made of cyclopropyl, cyclobutyl, cyclopentyl, and cyclohexyl. The cycloalkyl may be optionally substituted, e.g. with halogen, aryl, or heteroaryl.

In a preferred embodiment, the R³ is an alkenyl group, more preferably R³ is allyl.

In another preferred embodiment, R³ is an aryl group or an alkylene-aryl group, more preferably R³ is phenyl or benzyl, most preferably R³ is phenyl.

In yet another preferred embodiment, R² is H.

In yet another preferred embodiment R¹ is H or an alkyl group, more preferably R¹ is methyl.

Most preferably, the compound of general formula (I) is (1-fluoroethyl)allyl carbonate or (1-fluoroethyl)phenyl carbonate.

Another aspect of the present invention concerns a method for the manufacture of a compound of the general formula R1R2CF-O-C(O)-O-R3 comprising a step of reacting a fluoroformate of the general formula R1R2CF-O-C(O)F with an alcohol of the general formula R3-OH ; wherein R1 is H, F, alkyl, cycloalkyl, alkylene-aryl, or alkylene-heteroaryl ; wherein R2 is H, and wherein R3 is a double bond-containing group.

Yet another aspect of the present invention concerns a method for the manufacture of a compound of the general formula R1R2CF-O-C(O)-O-R3 comprising a first step of reacting phosgene or a phosgene analogue with a compound of the general formula R1R2CF-OH to form an intermediate of the general formula R1R2CF-O-C(O)X and a second step of reacting the intermediate of the general formula R1R2CF-O-C(O)X with a compound of the general formula R3-OH ; wherein R1 and R2 are independently H, F, alkyl, cycloalkyl, alkylene-aryl, or alkylene-heteroaryl ; wherein R3 is a double bond-containing group ; and wherein X- is a leaving group, preferably X- is chloride or fluoride.

Still another aspect of the present invention concerns method for the manufacture of a compound of the general formula R1R2CF-O-C(O)-O-R3 comprising a first step of reacting phosgene or a phosgene analogue with a compound of the general formula R3-OH to form an intermediate of the general formula R3-O-C(O)X and a second step of reacting the intermediate of the general formula R3-O-C(O)X with a compound of the general formula R1R2CF-OH, wherein R1 and R2 are independently H, F, alkyl, cycloalkyl, alkylene-aryl, or alkylene-heteroaryl ; wherein R3 is a double bond-containing group ; and wherein X- is a leaving group, preferably X- is chloride or fluoride.

"Phosgene analogue" is intended to denote a compound that is known in the art as a replacement for phosgene. Preferably, the phosgene analogue is difluorophosgene (FC(O)F), trichloromethyl chloroformate ("diphosgene"), bis(trichloromethyl) carbonate ("triphosgene"), S,S-dimethyl dithiocarbonate (DMDTC), carbonyldiimidazole (CDI), or N,N-diphenylurea.

The individual steps of the process according to the invention may be performed in the presence of a base, e.g. in the presence of a tertiary amine, e.g. triethylamine or pyridine. Alternatively, the steps can be performed in the absence of a base.

The individual steps of the process according to the invention are typically conducted in liquid phase, usually in the presence of a solvent, preferably a polar aprotic solvent. Examples of suitable solvents are acetonitrile, dimethylformamide (DMF), N-methyl-2-pyrrolidone (NMP), tetrahydrofurane, dichloromethane, toluene, CF₃-toluene and ionic liquids. Alternatively, the step can be performed in the absence of a solvent or - if the reaction is performed in the presence of a base - the base can serve as a solvent.

The reaction temperature during the individual steps is not critical. Often, the reaction is exothermic, thus, it may be advisable to cool the reaction mixture. The temperature is preferably equal to or higher than - 80°C, more preferably, equal to or higher than -78°C. The upper temperature can be dependent from pressure and boiling point of the starting materials. Often, the temperature is equal to or lower than 85°C. The reaction can be performed in any suitable reactor, e.g. in an autoclave. The reaction can be performed batch wise or continuously.

The resulting reaction mixture can be separated by known methods, e.g. by distillation, precipitation and/or crystallization. If desired, the reaction mixture can be contacted with water to remove water-soluble constituents.

A process for the manufacture of fluoroformates and of the specific example CH₃CHFC(O)F is described in WO 2011/006822.

Another aspect of the present invention is the use of a compound of a compound of the general formula R1R2CF-O-C(O)-O-R3 wherein R1 and R2 are independently H, F, alkyl, cycloalkyl, alkylene-aryl, or alkylene-heteroaryl ; and wherein R3 is a double bond-containing group as a solvent additive or as solvent for lithium ion batteries, lithium air batteries, lithium sulphur batteries, supercapacitors or hybrid supercapacitors.

Another aspect of the present invention concerns a solvent composition for lithium ion batteries, lithium air batteries, lithium sulfur batteries, supercapacitors or hybrid supercapacitors, comprising at least one solvent useful for lithium ion batteries, further comprising at least one of the compounds according to the invention.

Compounds of formula (I) are often applied in solvent compositions or in electrolyte compositions together with at least one suitable solvent known to the expert in the field of lithium ion batteries or supercapacitors. For example, organic carbonates, but also lactones, formamides, pyrrolidinones, oxazolidinones, nitroalkanes, N,N-substituted urethanes, sulfolane, dialkyl sulfoxides, dialkyl sulfites, acetates, nitriles, acetamides, glycol ethers, dioxolanes, dialkyloxyethanes, trifluoroacetamides, are very suitable as solvents.

Preferably, the aprotic organic solvent is selected from the group of dialkyl carbonates (which are linear) and alkylene carbonates (which are cyclic), ketones, and formamides. Dimethyl formamide, carboxylic acid amides, for example, N,N-dimethyl acetamide and N,N-diethyl acetamide, acetone, acetonitrile, dimethyl carbonate, diethyl carbonate, methyl ethyl carbonate, cyclic alkylene carbonates, e.g. ethylene carbonate, propylene carbonate, and vinylidene carbonate, are suitable solvents.

Fluorosubstituted compounds different from the compounds of formula (I) mentioned above, for example, fluorinated carbonic esters which are selected from the group of fluorosubstituted ethylene carbonates, polyfluorosubstituted dimethyl carbonates, fluorosubstituted ethyl methyl carbonates, and fluorosubstituted diethyl carbonates are other solvents or, preferably, suitable additional additives in the electrolytic compositions. Preferred fluorosubstituted carbonates are monofluoroethylene carbonate, 4,4-difluoro ethylene carbonate, 4,5-difluoro ethylene carbonate, 4-fluoro-4-methyl ethylene carbonate, 4,5-difluoro-4-methyl ethylene carbonate, 4-fluoro-5-methyl ethylene carbonate, 4,4-difluoro-5-methyl ethylene carbonate, 4-(fluoromethyl)-ethylene carbonate, 4-(difluoromethyl)-ethylene carbonate, 4-(trifluoromethyl)-ethylene carbonate, 4-(fluoromethyl)-4-fluoro ethylene carbonate, 4-(fluoromethyl)-5-fluoro ethylene carbonate, 4-fluoro-4,5-dimethyl ethylene carbonate, 4,5-difluoro-4,5-dimethyl ethylene carbonate, and 4,4-difluoro-5,5-dimethyl ethylene carbonate ; dimethyl carbonate derivatives including fluoromethyl methyl carbonate, difluoromethyl methyl carbonate, trifluoromethyl methyl carbonate, bis(difluoro)methyl carbonate, and bis(trifluoro)methyl carbonate ; ethyl methyl carbonate derivatives including 2-fluoroethyl methyl carbonate, ethyl fluoromethyl carbonate, 2,2-difluoroethyl methyl carbonate, 2-fluoroethyl fluoromethyl carbonate, ethyl difluoromethyl carbonate, 2,2,2-trifluoroethyl methyl carbonate, 2,2-difluoroethyl fluoromethyl carbonate, 2-fluoroethyl difluoromethyl carbonate, and ethyl trifluoromethyl carbonate ; and diethyl carbonate derivatives including ethyl (2-fluoroethyl) carbonate, ethyl (2,2-difluoroethyl) carbonate, bis(2-fluoroethyl) carbonate, ethyl (2,2,2-trifluoroethyl) carbonate, 2,2-difluoroethyl 2'-fluoroethyl carbonate, bis(2,2-difluoroethyl) carbonate, 2,2,2-trifluoroethyl 2'-fluoroethyl carbonate, 2,2,2-trifluoroethyl 2',2'-difluoroethyl carbonate, and bis(2,2,2-trifluoroethyl) carbonate, 4-fluoro-4-vinylethylene carbonate, 4-fluoro-5-vinylethylene carbonate, 4,4-difluoro-4-vinylethylene carbonate, 4,5-difluoro-4-vinylethylene carbonate, 4-fluoro-4,5-divinylethylene carbonate, 4,5-difluoro-4,5-divinylethylene carbonate, 4-fluoro-4-phenylethylene carbonate, 4-fluoro-5-phenylethylene carbonate, 4,4-difluoro-5-phenylethylene carbonate, 4,5-difluoro-4-phenylethylene carbonate and 4,5-difluoro-4,5-diphenylethylene carbonate, fluoromethyl phenyl carbonate, 2-fluoroethyl phenyl carbonate, 2,2-difluoroethyl phenyl carbonate and 2,2,2-trifluoroethyl phenyl carbonate, fluoromethyl vinyl carbonate, 2-fluoroethyl vinyl carbonate, 2,2-difluoroethyl vinyl carbonate and 2,2,2-trifluoroethyl vinyl carbonate, fluoromethyl allyl carbonate, 2-fluoroethyl allyl carbonate, 2,2-difluoroethyl allyl carbonate and 2,2,2-trifluoroethyl allyl carbonate.

Other suitable additional additives useful in the electrolyte compositions according to the present invention are those described in WO2007/042471.

The solvent composition or electrolyte composition may also additionally contain benzene, fluorobenzene, toluene, trifluorotoluene, xylene or cyclohexane.

Partially fluorinated esters suitable as solvent or additional solvent additive are for example those described in US 6,677,085 partially fluorinated compound derived from a diol corresponding to the formula R¹CO-O-[CHR³(CH₂)ₘ-O]ₙ -R² wherein R¹ is a (C1 - C8) alkyl group or a (C3 - C8) cycloalkyl group, wherein each of said groups is partially fluorinated or perfluorinated so that at least one hydrogen atom of the group is replaced by fluorine ;R² is a (C1 - C8) alkyl carbonyl or (C3 - C8) cycloalkyl carbonyl group, wherein said alkylcarbonyl or cycloalkylcarbonyl group may optionally be partially fluorinated or perfluorinated ; R³ is a hydrogen atom or a (C1 - C8) alkyl or (C3 - C8) cycloalkyl group ; m is 0, 1, 2 or 3, and n is 1, 2 or 3.

Another aspect of the present invention concerns an electrolyte composition for lithium ion batteries, lithium air batteries, lithium sulfur batteries, supercapacitors or hybrid supercapacitors, comprising at least one compound according to the invention, at least one solvent useful for lithium ion batteries or supercapacitors and at least one electrolyte salt.

The electrolyte composition, further to the at least one compound of general formula (I), comprises at least one dissolved electrolyte salt. Such salts have the general formula MₐA_{b}. M is a metal cation, and A is an anion. The overall charge of the salt MₐA_{b} is 0. M is preferably selected from Li⁺ and NR₄⁺. Preferred anions are PF₆⁻, PO₂F₂⁻, AsF₆⁻, BF₄⁻, ClO₄⁻, N(CF₃SO₂)₂⁻ and N(i-C₃F₇SO₂)₂⁻.

Preferably, M is Li⁺. Especially preferably, M is Li⁺ and the solution comprises at least one electrolyte salt selected from the group consisting of LiB_{F}4, LiClO₄, LiAsF₆, LiPF₆, LiPO₂F₂, LiN(CF₃SO₂)₂ and LiN(i-C₃F₇SO₂)₂. Lithium bis(oxalato)borate can be applied as an additional additive. The concentration of the electrolyte salt is preferably between 0.8 and 1.2 molar, more preferably 1.0 molar. Often, the electrolyte composition may comprise LiPF₆ and LiPO₂F₂.

If LiPO₂F₂ is the only electrolyte salt, its concentration in the electrolyte composition is, as mentioned, preferably between 0.9 and 1.1 molar, more preferably 1 molar. If LiPO₂F₂ is applied as an additive together with another electrolyte salt, especially together with LiPF₆, the concentration of LiPO₂F₂ in the electrolyte composition preferably is equal to or greater than 0.1 % by weight, more preferably equal to or greater than 0.5 % by weight ; preferably, its concentration is equal to or lower than 10 % by weight, more preferably, equal to or lower than 5 % by weight when the total electrolyte composition including electrolyte salt, solvent and additives is set as 100 % by weight.

The compounds of formula (I) can be introduced into the electrolyte composition separately or in the form of a mixture with other compounds, e.g. as a mixture with one or more solvents used in the electrolyte composition or together with the electrolyte salt or together with other additives.

Still another aspect of the present invention are lithium ion batteries, lithium air batteries and lithium sulfur batteries comprising a solvent composition as outlined above or an electrolyte composition as outlined above.

The compounds according to this invention may advantageously be used as a solvent, a solvent additive or a co-solvent in a concentration from 1 to 15 wt %, preferably from 3 to 10 wt %, more preferably between 4 and 6 wt % and most preferably around 5 wt % relative to the total weight of the electrolyte composition.

Accordingly, another aspect of the invention concerns the use of a compound according to this invention in an electrolyte composition, in an electrolyte composition for Li ion batteries, Li air batteries or Li sulfur batteries, wherein the concentration of the compound according to any one of the claims 1 to 7 is from 1 to 15 wt %, preferably from 3 to 10 wt %, more preferably between 4 and 6 wt % and most preferably around 5 wt % ; relative to the total weight of the electrolyte composition.

In another aspect of the invention, the lithium ion batteries comprises an anode, preferably an anode made from carbon comprising a copper foil, a cathode, preferably a cathode made from lithium metal oxides comprising an aluminum foil, a separator, preferably a separator made from an insulating polymer, and a solvent composition or an electrolyte composition as described above. The foils used for anode and cathode are also called current collectors.

Yet another aspect of the present invention are supercapacitors or hybrid supercapacitors comprising a solvent composition as outlined above or an electrolyte composition as outlined above.

Should the disclosure of any patents, patent applications, and publications which are incorporated herein by reference conflict with the description of the present application to the extent that it may render a term unclear, the present description shall take precedence.

The invention will now be further described in examples without intending to limit it.

### Examples :

### Example 1 : Synthesis of allyl 1-fluoroethyl carbonate

A 2.5 1 perfluoroalkoxylalkane reactor equipped with a temperated double mantle, a reflux condenser and a mechanical stirrer was charged with 1023 g 1-fluoroethyl fluoroformate (prepared as described in WO 2011/006822). After cooling the material to 3°C, a mixture of 231 g pyridine and 517 g allylic alcohol was slowly added over a period of 2 hours. The reaction temperature was kept below 60°C. After cooling to room temperature, the mixture was washed twice with citric acid solution (30 % in deionized water). The product was obtained as a colourless liquid (yield : 91 %, GC purity > 95 %. The product can optionally be further purified by distillation to obtain the product in a GC purity > 99.9 %.

### Example 2 : Synthesis of 1-fluoroethyl phenyl carbonate

A 2.5 1 perfluoroalkoxylalkane reactor equipped with a temperated double mantle, a reflux condenser and a mechanical stirrer was charged with 1007 g 1-fluoroethyl fluoroformate (prepared as described in WO 2011/006822). After cooling the material to 3°C, a mixture of 238 g pyridine and 819 g phenol was slowly added over a period of 2 hours. The reaction temperature was kept below 45°C. After cooling to room temperature, the mixture was washed three times with citric acid solution (30 % in deionized water). The product was obtained as a colourless liquid (yield : 81 %, GC purity > 94 %). The product can optionally be purified by distillation and/or crystallization to obtain a product in a GC purity > 99.9 %.

### Example 3 : Performance testing

Test system : Pouch full cell consisting of: [LiCoO₂ : Super-P^{®} (conductive carbon black obtainable from MMM Carbon, Belgium) : PVdF (Solef^{®} 5130 from Solvay Specialty Polymers) binder = 92 : 4 : 4 (wt. %)] as positive electrode and [SCMG-AR^{®} (artificial graphite obtainable from Showa Denko) : Super-P^{®} (conductive carbon black obtainable from MMM Carbon, Belgium) : PVdF (Solef^{®} 5130 from Solvay Specialty Polymers) binder = 90 : 4 : 6 (wt. %)] as negative electrode. Polyethylene was used as separator. A standard electrolyte composition [(1.0M LiPF₆ / ethylene carbonate + dimethyl carbonate (1:2 (v/v)] was used to which the fluorinated additives according to the invention were added under dry room atmosphere.

The preparation of the pouch cells consisted of the following steps in that order : (1) mixing, (2) coating & Drying (3) pressing, (4) slitting, (5) Tap welding, (6) Winding, (7) Jelly roll pressing, (8) Jelly roll taping, (9) Pouch forming, (10) Pouch 2-side sealing, (11) Electrolyte filling, and (12) Vacuum sealing.

For the Cycle Performance, 300 cycles were performed under C-rate of 1.0.

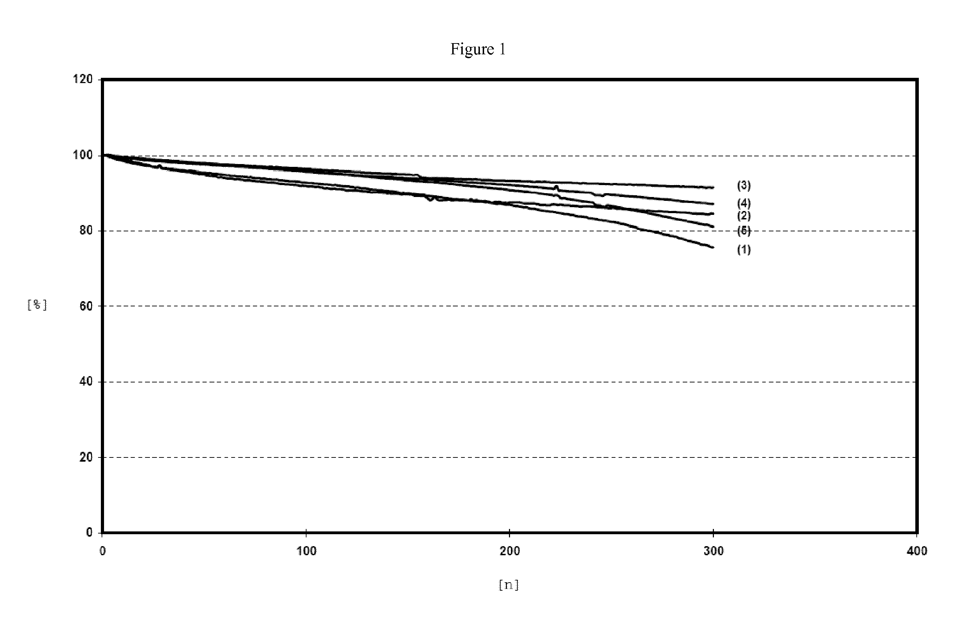

Figure 1 shows the unexpected advantageous effect of allyl 1-fluoroethyl carbonate in different concentrations (x-axis : cycle number, y-axis : efficiency [%]). After 300 cycles, use of an electrolyte composition comprising 3, 5, 7.5 and 10 wt % resulted in a residual efficiency of 84.4 %, 91.5 %, 87.1 %, 81.1 %, respectively. In a comparative example, use of the standard electrolyte composition resulted in a residual efficiency of 75.6 %.

## Claims

1. A compound of general formula (I),
R¹R²CF-O-C(O)-O-R³
wherein R¹ and R² are independently H, F, alkyl, cycloalkyl, alkylene-aryl, or alkylene-heteroaryl ; and
wherein R³ is a double bond-containing group.

2. The compound according to claim 1 wherein R³ is an alkenyl, preferably R³ is allyl.

3. The compound according to claim 1 wherein R³ is an aryl group or an alkylene-aryl group, preferably R³ is phenyl or benzyl, most preferably R³ is phenyl.

4. The compound according to any one of claims 1 to 3 wherein R² is H.

5. The compound according to any one of claims 1 to 4 wherein R¹ is H or an alkyl group, preferably R¹ is methyl.

6. The compound according to any one of the claims 1 to 5 wherein R1 is methyl, R2 is H, R3 is allyl and the compound is (1-fluoroethyl)allyl carbonate.

7. The compound according to any one of the claims 1 to 5 wherein R1 is methyl, R2 is H, R3 is phenyl and the compound is (1-fluoroethyl)phenyl carbonate.

8. A method for the manufacture of a compound of general formula (I),
R¹R²CF-O-C(O)-O-R³
comprising a step of reacting a fluoroformate of general formula (II),
R¹R²CF-O-C(O)F
with an alcohol of general formula (III), R³-OH ;
wherein R¹ is H, F, alkyl, cycloalkyl, alkylene-aryl, or alkylene-heteroaryl ;
wherein R² is H, and
wherein R³ is a double bond-containing group.

9. A method for the manufacture of a compound of general formula (I),
R¹R²CF-O-C(O)-O-R³
comprising a first step of reacting phosgene or a phosgene analogue with a compound of the general formula (IV) R¹R²CF-OH to form an intermediate of the general formula (V)
R¹R²CF-O-C(O)X
and a second step of reacting the intermediate of the general formula (V) with a compound of the general formula (VI) R³-OH ;
wherein R¹ and R² are independently H, F, alkyl, cycloalkyl, alkylene-aryl, or alkylene-heteroaryl ;
wherein R³ is a double bond-containing group ; and
wherein X⁻ is a leaving group, preferably X⁻ is chloride or fluoride.

10. A method for the manufacture of a compound of general formula (I),
R¹R²CF-O-C(O)-O-R³
comprising a first step of reacting phosgene or a phosgene analogue with a compound of the general formula (VI) R³-OH to form an intermediate of the general formula (VII)
R³-O-C(O)X
and a second step of reacting the intermediate of the general formula (VII) with a compound of the general formula (VIII)) R¹R²CF-OH,
wherein R¹ and R² are independently H, F, alkyl, cycloalkyl, alkylene-aryl, or alkylene-heteroaryl ;
wherein R³ is a double bond-containing group ; and
wherein X⁻ is a leaving group, preferably X⁻ is chloride or fluoride.

11. Use of a compound of a compound of general formula (I),
R¹R²CF-O-C(O)-O-R³
wherein R¹ and R² are independently H, F, alkyl, cycloalkyl, alkylene-aryl, or alkylene-heteroaryl ; and
wherein R³ is a double bond-containing group ;
as a solvent additive or as solvent for lithium ion batteries, lithium air batteries, lithium sulphur batteries, supercapacitors or hybrid supercapacitors.

12. A solvent composition for lithium ion batteries, lithium air batteries, lithium sulfur batteries, supercapacitors or hybrid supercapacitors, comprising at least one solvent useful for lithium ion batteries, further comprising at least one of the compounds according to any one of the claims 1 to 7.

13. An electrolyte composition for lithium ion batteries, lithium air batteries, lithium sulfur batteries, supercapacitors or hybrid supercapacitors, comprising at least one of the compounds according to any one of the claims 1 to 7, at least one solvent useful for lithium ion batteries or supercapacitors and at least one electrolyte salt.

14. A lithium ion battery, a lithium air battery, a lithium sulfur battery, a supercapacitor or a hybrid supercapacitor containing at least one of the compounds according to any one of the claims 1 to 7.

15. Use of the compound according to any one of the claims 1 to 7 as a component in an electrolyte composition, in an electrolyte composition for Li ion batteries, Li air batteries or Li sulfur batteries, wherein the concentration of the compound according to any one of the claims 1 to 7 is from 1 to 15 wt %, preferably from 3 to 10 wt %, more preferably between 4 and 6 wt % and most preferably around 5 wt % ; relative to the total weight of the electrolyte composition.
